# EUROPÄISCHE PATENTANMELDUNG

(11) **EP 4 817 652 A1**
(43) Veröffentlichungstag der Anmeldung: **07.10.2026**
(21) Anmeldenummer: 25167838.9
(22) Anmeldetag: 01.04.2025
(51) Int. Cl.: A61M 5/142, A61M 39/28, G01N 29/44

(54) **MEDIZINISCHE VORRICHTUNG ZUR ERKENNUNG, INSBESONDERE PRÄSENZERKENNUNG, UND/ODER KLASSIFIZIERUNG EINES MEDIZINISCHEN ARTIKELS**

(71) Anmelder: B. Braun Melsungen AG, 34212 Melsungen (DE)
(72) Erfinder: VOGEL, Markus, 37191 Katlenburg-Lindau (DE); SCHWARZ, Jan, 34212 Melsungen (DE); JANOSCHKA, Tobias, 34125 Kassel (DE); KLINK, Florian, 34131 Kassel (DE); SIELEMANN, Martin, 34466 Wolfhagen (DE)
(74) Vertreter: Winter, Brandl - Partnerschaft mbB

(57) **Zusammenfassung**

Die Offenbarung betrifft eine medizinische Vorrichtung (10), die zur Erkennung, insbesondere Präsenzerkennung, und/oder Klassifizierung eines medizinischen Artikels (11) eingerichtet ist, umfassend: eine Anregungseinheit (12), die eingerichtet ist, einen medizinischen Artikel (11) mit zumindest einer Anregung anzuregen, sodass dieser in Schwingung versetzt wird; eine Erfassungseinheit (13), die eingerichtet ist, eine Schallemission (16) des angeregten medizinischen Artikels zu erfassen; und eine Auswerteeinheit (14), die eingerichtet ist, durch eine Analyse der Schallemission (16) den medizinischen Artikel (11) zu erkennen und/oder zu klassifizieren, vorzugsweise zu erkennen, ob der medizinische Artikel (11) mit zumindest einem vorbestimmten medizinischen Artikel übereinstimmt.

## Beschreibung

### Technisches Gebiet

Die vorliegende Offenbarung betrifft eine medizinische Vorrichtung, die zur Erkennung, insbesondere Präsenzerkennung, und/oder Klassifizierung eines medizinischen Artikels eingerichtet ist, sowie ein Verfahren zur Erkennung eines medizinischen Artikels mit einer solchen Vorrichtung.

### Technischer Hintergrund

Die Dosierung medizinischer Infusionsflüssigkeiten und deren Zuführung zu einem Blutgefäß eines Patienten kann über eine Infusionspumpe erfolgen. Ein mögliches Wirkprinzip einer solchen Infusionspumpe ist, einem Infusionsleitungs- bzw. - schlauchsystem, durch das die Infusionsflüssigkeit geleitet wird, mittels einer Verdrängereinheit der Infusionspumpe eine peristaltische Bewegung aufzuprägen, wodurch die Infusionsflüssigkeit von einem Infusionsbehälter hin zu einem Zugang am Patienten gefördert wird. Ein Infusionsschlauchsystem ist oft als Einmalartikel ausgestaltet. Um sicherzustellen, dass ein Fluss der Infusionsflüssigkeit zum Patienten nur bei Bedarf, das heißt nur im Betrieb der Infusionspumpe erfolgt, ist ein Klemmensystem üblich, über das der Infusionsschlauch abgesperrt werden kann.

Eine bekannte Infusionspumpe weist ein Klemmenmodul zur Aufnahme einer als Schiebeklemme ausgebildeten Schlauchklemme auf. Das Klemmenmodul weist einen Photodetektor zur Erkennung der Farbe der Schiebeklemme auf. Mit dieser Farbe ist einer von verschiedenen medizinischen Fachbereichen bzw. Anwendungsbereichen des eingelegten Infusionsleitungssystems definiert, an dem die Schiebeklemme unverlierbar befestigt ist. Die Infusionspumpe kann dann etwa auf Grund des über die Farbe erkannten Anwendungsbereiches eine Voreinstellung vornehmen, also z.B. auf einem Display passende Funktionen anbieten.

Über eine Erkennung, insbesondere Präsenzerkennung, und/oder Klassifizierung eines medizinischen Artikels wie etwa ein Infusionsleitungssystem über einen Photodetektor, der die Farbe einer Schiebeklemme erkennt, kann jedoch eine Verwendung von Nichtoriginalprodukten, welche gegebenenfalls die beabsichtigten bzw. gewünschten Funktionen nicht bestmöglich ausführen und potentiell einen Patienten gefährden können, nicht ausgeschlossen werden.

Es hat sich daher herausgestellt, dass ein Bedarf besteht, eine alternative, insbesondere verbesserte, sichere und effiziente medizinische Vorrichtung, die zur Erkennung, insbesondere Präsenzerkennung, und/oder Klassifizierung eines medizinischen Artikels eingerichtet ist, bereitzustellen.

### Zusammenfassung der vorliegenden Offenbarung

Es ist daher die Aufgabe der vorliegenden Offenbarung, die Nachteile aus dem Stand der Technik zu vermeiden oder wenigstens zu verringern und insbesondere eine alternative, bevorzugt verbesserte, medizinische Vorrichtung, die zur Erkennung, insbesondere Präsenzerkennung, und/oder Klassifizierung eines medizinischen Artikels eingerichtet ist, bereitzustellen, die eine sichere und effiziente Erkennung eines medizinischen Artikels ermöglicht.

Die Aufgabe der vorliegenden Offenbarung wird durch eine medizinische Vorrichtung mit den Merkmalen des Anspruchs 1 sowie durch ein Verfahren zur Erkennung eines medizinischen Artikels mit einer solchen Vorrichtung mit den Merkmalen des nebengeordneten Anspruchs 13 gelöst. Vorteilhafte Ausgestaltungen sind Gegenstand der Unteransprüche und/oder werden nachfolgend erläutert.

Ein erster Aspekt der vorliegenden Offenbarung betrifft eine medizinische Vorrichtung, die zur Erkennung, insbesondere Präsenzerkennung, und/oder Klassifizierung eines medizinischen Artikels eingerichtet ist, umfassend: eine Anregungseinheit, die eingerichtet ist, einen medizinischen Artikel mit zumindest einer Anregung anzuregen, sodass dieser in Schwingung versetzt wird, eine Erfassungseinheit, die eingerichtet ist, eine Schallemission des angeregten medizinischen Artikels zu erfassen, und eine Auswerteeinheit, die eingerichtet ist, durch eine Analyse der Schallemission den medizinischen Artikel zu erkennen und/oder zu klassifizieren, vorzugsweise zu erkennen, ob der medizinische Artikel mit zumindest einem vorbestimmten medizinischen Artikel übereinstimmt.

Der Begriff medizinische Vorrichtung meint vorliegend insbesondere ein Gerät, das neben der Erkennungsfunktion auch noch eine andere medizinische Funktion ausführen kann. Beispielsweise kann die medizinische Vorrichtung eine Infusionspumpe, ein Dialysegerät oder dergleichen umfassen oder sein. Die medizinische Vorrichtung kann vorzugsweise eingerichtet sein, mit dem medizinischen Artikel betrieben zu werden. Beispielsweise kann die Infusionspumpe mit einem Infusionsleitungssystem, welches eine Schiebeklemme aufweist, betrieben werden. Die medizinische Vorrichtung kann vorzugsweise eingerichtet sein, ein Warnsignal auszugeben, wenn der medizinische Artikel nicht mit einem vorbestimmten medizinischen Artikel übereinstimmt.

Unter einer Erkennung wird vorliegend ein Feststellen eines bestimmten medizinischen Artikels, beispielsweise eines bestimmten Infusionsleitungssystems, verstanden. Beispielsweise wird eine bestimmte Schiebeklemme eines bestimmten Infusionsleitungssystems erkannt. Der vorbestimmte medizinische Artikel meint beispielsweise eben eine Schiebeklemme eines Infusionsleitungssystems oder das Infusionsleitungssystem selbst.

Unter Präsenzerkennung wird vorliegend ein Feststellen eines Vorliegens irgendeines medizinischen Artikels verstanden. Beispielsweise wird erkannt, dass ein medizinischer Artikel in oder an der medizinischen Vorrichtung angeordnet ist oder eben nicht.

Unter Klassifizierung wird vorliegend ein Feststellen einer Zugehörigkeit eines medizinischen Artikels zu einer Gruppe dieser medizinischen Artikel verstanden. Beispielsweise wird erkannt, dass es sich um eine bestimmte Schiebeklemme und nicht etwa um einen anderen medizinischen Artikel wie etwa eine Spritze, einen Schlauch, einen Filter, etc. handelt.

Ein vorbestimmter medizinischer Artikel meint vorliegend vorzugsweise einen medizinischen Artikel, der mit der medizinischen Vorrichtung verwendet werden soll, beispielsweise eine spezielle proprietäre Schiebeklemme eines Infusionsleitungssystems für eine Infusionspumpe. Der offenbarungsgemäße medizinische Artikel kann grundsätzlich jedoch ein beliebiger Einmal- oder Mehrwegartikel sein. Wenn die medizinische Vorrichtung ein Dialysegerät ist, kann der medizinische Artikel beispielsweise auch ein Dialysator sein.

Der Begriff Anregungseinheit meint vorliegend insbesondere eine strukturelle Einheit, die eingerichtet ist, einen medizinischen Artikel mit einer Anregung anzuregen. Die Anregungseinheit kann einteilig oder mehrteilig ausgeführt sein. Die Anregungseinheit kann beispielsweise an der Infusionspumpe angeordnet sein. Die Anregungseinheit kann beispielsweise einen Aktuator oder einen Lautsprecher umfassen. Die Anregung kann eine mechanische Anregung und/oder eine akustische Anregung umfassen. Die Anregung kann beliebige Signale umfassen. Durch die Anregung kann der medizinische Artikel in Schwingung versetzt werden. Die Schwingung führt zu einer Schallemission, beispielsweise Körperschall oder Hörschall. Die Schwingung ist eine Antwort des medizinischen Artikels auf die Anregung.

Der Begriff Erfassungseinheit meint vorliegend eine strukturelle Einheit, die eingerichtet ist, eine Schallemission des angeregten medizinischen Artikels zu erfassen. Die Erfassungseinheit kann einteilig oder mehrteilig ausgeführt sein. Die Erfassungseinheit kann beispielsweise ein Mikrofon für Hörschallmessungen aufweisen oder einen Beschleunigungssensor für Körperschallmessungen.

Der Begriff Auswerteeinheit meint vorliegend eine Einheit, die eingerichtet ist, auf Basis der erfassten Schallemission den medizinischen Artikel zu erkennen und/oder zu klassifizieren und/oder zu erkennen, ob ein medizinischer Artikel präsent ist oder nicht. Die Auswerteeinheit kann einteilig oder mehrteilig ausgeführt sein. Die Auswerteeinheit kann in der medizinischen Vorrichtung integriert sein oder separat als physisch bzw. räumlich getrennte Einheit ausgeführt sein. Die Auswerteeinheit kann Hardware und Software umfassen. Die Auswerteinheit kann beispielsweise einen Mikroprozessor umfassen. Die Auswerteeinheit kann vorzugsweise die Anregung und die zugehörige Schallemission auswerten und mit einem Vergleichsbeispiel vergleichen, um den medizinischen Artikel zu erkennen und/oder zu klassifizieren.

Der Offenbarung liegt die Erkenntnis zugrunde, dass medizinische Artikel von Wettbewerbern kopiert werden. Neben dem Schutz vor unlauterem Wettbewerb können weiterhin mangelhafte Kopien zu schlechteren Behandlungsergebnissen führen. Die Offenbarung schlägt hier eine Erkennungsmöglichkeit für spezielle medizinische Artikel durch die medizinische Vorrichtung vor. Die medizinische Vorrichtung macht sich den Umstand zu eigen, dass jedes strukturelle Bauteil ein eigenes Schwingungsverhalten aufweist, sprichwörtlich einen schwingungsbasierten bzw. audiospektralen Fingerabdruck. Die medizinische Vorrichtung regt den medizinischen Artikel mit einer definierten Anregung an und misst die Schallemission bzw. die Antwort des medizinischen Artikels und wertet die Antwort und gegebenenfalls die Anregung aus, um zu erkennen, ob der richtige medizinische Artikel mit der medizinischen Vorrichtung verwendet wird.

Auf diese Weise kann sichergestellt werden, dass der richtige medizinische Artikel verwendet wird. Dies kann sich vorteilhaft auf die Verlässlichkeit der Anwendung der medizinischen Vorrichtung auswirken. Dies kann sich außerdem vorteilhaft auf die Qualität und Sicherheit der medizinischen Vorrichtung auswirken. Auf diese Weise können vorteilhaft Konkurrenzprodukte mit geringerer Qualität identifiziert werden. Dadurch kann es vorteilhaft möglich sein, die medizinischen Artikel sicher und eindeutig zu erkennen.

In anderen Worten ausgedrückt handelt es sich um eine Detektionsmethodik zur Präsenzerkennung, sowie eine Klassifizierung von medizinischen Ein- oder Mehrwegartikel. Als Anwendungsgebiet wird hier stellvertretend auf ein Infusionspumpensystem eingegangen. Mit Hilfe der Anregung durch eine oder mehrere Schallquellen und einer oder mehrerer schall-/körperschallrückgekoppelten Messungen (Frequenz, Amplitude, Phase und/oder Spektrum), wird ein Verbrauchsartikel erkannt und kann anschließend klassifiziert und/oder erkannt werden. Alternativ zu einer Anregung durch Schallquellen ist eine mechanische Anregung möglich. Dies passiert nach dem Prinzip eines "akustischen Fingerabdrucks" des jeweiligen Verbrauchsartikels. Dieser akustische Fingerabdruck des Verbrauchsartikels wird durch Materialeigenschaften und/oder Formgebung (z.B. Hohlstrukturen) erzielt. Die Klassifizierung kann per klassischen Algorithmus, datenzentrisches Modell (z.B. neuronales Netzwerk) oder durch Auswertung vom Frequenzgang erfolgen. Zum Beispiel kann die Klassifizierung durch Komparatoren einer diskret aufgebauten elektronischen Schaltung erfolgen.

Gemäß einer bevorzugten Ausführungsform kann die Analyse eine Auswertung einer oder mehrerer der folgenden Größen umfassen: eine oder mehrere Frequenzen, eine oder mehrere Amplituden, eine oder mehrere Phasenverschiebungen.

Die Auswertung der Schallemission kann beispielsweise mit mathematischen Methoden, wie beispielsweiser einer Fast Fourier Transformation, erfolgen. Die Auswertung kann im Zeitbereich und/oder im Frequenzbereich erfolgen. Die Auswertung kann die Analyse des Frequenzgangs umfassen. Bei einer Anregung mit einer einzelnen Frequenz und gleichbleibender Amplitudenstärke kann beispielsweise die Phasenverschiebung der Schallemission und deren Amplitude in Relation zur Anregung analysiert werden. Diese kennzeichnet den medizinischen Artikel. Die so ermittelte Phasenverschiebung und/oder Amplitude des medizinischen Artikels kann dann beispielsweise mit Vergleichsdaten zumindest eines vorbestimmten medizinischen Artikels einer Datenbank verglichen werden. Bei entsprechender hoher Übereinstimmung kann dann ein medizinischer Artikel, beispielsweise die Schiebeklemme mit der Produktnummer 1234 erkannt werden. Hierzu kann beispielsweise für den medizinischen Artikel im Vorfeld für die gleichen Versuchsbedingungen, u.a. gleiche Anordnung medizinischer Artikel in medizinischer Vorrichtung, gleiche Anregung, eine Schallemission aufgenommen werden und auf die gleiche Weise hinsichtlich Phasenverschiebung und Amplitude ausgewertet werden. Die beiden Kennzahlen, Phasenverschiebung und Amplitude können beispielsweise noch mit einem ersten Toleranzband und/oder zweiten Toleranzband beaufschlagt werden. Führt eine Auswertung einer Schallemission zu einer Amplitude und/oder Phasenverschiebung innerhalb des ersten Toleranzbandes (bspw. +- 1 %) wird von dem gleichen medizinischen Artikel ausgegangen. Führt eine Auswertung einer Schallemission zu einer Amplitude und/oder Phasenverschiebung innerhalb des zweiten Toleranzbandes (bspw. +- 2%) und außerhalb des ersten Toleranzbandes wird von einem medizinischen Artikel der gleichen Klasse ausgegangen, beispielweise eine Schiebeklemme, aber nicht der richtigen Schiebeklemme, ausgegangen. Wird beispielsweise der medizinische Artikel mit einem Stoß bzw. Dirac Impuls angeregt, können in der Schallemission durch die Auswertung eine oder mehrere charakteristische Frequenzen bzw. Eigenfrequenzen ermittelt werden. Hier kann ebenfalls durch eine Vergleichsmessung unter gleichen Versuchsbedingungen ein entsprechendes Frequenzspektrum für einen vorbestimmten medizinischen Artikel im Vorfeld erstellt und ausgewertet werden. Hierzu können eine oder mehrere Frequenzen und/oder zugehörige Amplituden ermittelt werden. Diese können ebenfalls wie oben beschrieben mit einem ersten Toleranzband und/oder einem zweiten Toleranzband beaufschlagt werden. Die Auswertung kann dann analog zur Auswertung der Phasenverschiebung erfolgen.

Auf diese Weise kann vorteilhaft präzise ein medizinischer Artikel erkannt oder klassifiziert werden.

Gemäß einer bevorzugten Ausführungsform kann die Analyse einen Vergleich mit zumindest einem Klassifizierungsmuster des zumindest einen vorbestimmten medizinischen Artikels umfassen.

Der Begriff Klassifizierungsmuster meint vorliegend zumindest eine Kennzahl wie beispielsweise Frequenz, Amplitude, Phasenverschiebung aus einer Vergleichsmessung für einen vorbestimmten medizinischen Artikel. Beispielweise wurde für zumindest einen medizinischen Artikel, beispielsweise eine Schiebeklemme, eine Vergleichsmessung durchgeführt und die Schallemission hinsichtlich Amplitude, Frequenz und/oder Phasenverschiebung ausgewertet und gegebenenfalls mit zumindest einem Toleranzband beaufschlagt.

Auf diese Weise kann mit ausreichend hoher Genauigkeit ein bestimmter medizinischer Artikel bestimmt werden.

Gemäß einer bevorzugten Ausführungsform kann die Analyse auf Basis eines Machine Learning Modells erfolgen, wobei das Machine Learning Modell für den zumindest einen vorbestimmten medizinischen Artikel trainiert wurde.

Das Machine Learning Modell kann vorzugsweise speziell für den zumindest einen vorbestimmten medizinischen Artikel trainiert werden. Beispielweise wurde ein Machine Learning Modell für eine Schiebeklemme mit einer Vielzahl an unterschiedlichen Messdurchläufen und/oder einer Vielzahl an unterschiedlichen Schiebeklemmen des gleichen Typs trainiert. Ein so trainiertes Machine Learning Modell kann vorteilhafterweise eine höhere Präzision bei der Erkennung von medizinischen Artikeln liefern als beispielsweise ein Vergleich mit einem Klassifizierungsmuster.

Gemäß einer bevorzugten Ausführungsform kann die Anregungseinheit eingerichtet sein, den medizinischen Artikel mechanisch anzuregen und/oder akustisch anzuregen.

Die mechanische Anregung kann vorteilhafterweise eine stärkere Anregung des medizinischen Artikels ermöglichen. Hierbei kann beispielsweise mit einem Vibrationsmotor der medizinische Artikel in einer Frequenz angeregt werden. Die akustische Anregung kann vorteilhafterweise berührungslos durch beispielsweise einen Lautsprecher erfolgen. Die mechanische Anregung kann beispielsweise mittelbar über ein Koppelelement erfolgen, das zwischen dem medizinischen Artikel und der Anregungseinheit angeordnet ist.

Gemäß einer bevorzugten Ausführungsform kann die Anregungseinheit eine oder mehrere der folgenden Komponenten umfassen: Vibrationsmotor, Aktuator, Lautsprecher.

Der Vibrationsmotor kann vorteilhaft eine mechanische Anregung mit einer Frequenz ermöglichen. Beispielsweise können auch zwei oder mehr Vibrationsmotoren den medizinischen Artikel an unterschiedlichen Stellen mit unterschiedlichen Frequenzen anregen. Der Aktuator kann beispielsweise einen Stößel umfassen, der einen Impuls bzw. Dirac Impuls auf den medizinischen Artikel einleitet, sodass das gesamte Frequenzspektrum angeregt wird. Der Lautsprecher kann vorteilhaft mit einer Frequenz oder mit einer Vielzahl an Frequenzen bzw. einem breitbandigen Frequenzsignal ähnlich einem weißen Rauschen den medizinischen Artikel anregen.

Je nach Beschaffenheit des medizinischen Artikels können unterschiedliche Komponenten der Anregungseinheit besser oder schlechter für die Anregung geeignet sein. Auf diese Weise kann vorteilhaft die Flexibilität und die Präzision der Vorrichtung erhöht werden.

Gemäß einer bevorzugten Ausführungsform kann die Anregungseinheit eingerichtet sein, den medizinischen Artikel mit einem oder mehreren der folgenden Signale anzuregen: Dirac Impuls, eine Frequenz, mehrere Frequenzen, breitbandiges Frequenzsignal.

Je nach Beschaffenheit des medizinischen Artikels kann es vorteilhaft sein, diesen mit einem oder sogar mehreren der oben aufgeführten Signale anzuregen, wenn dies zu einer besser unterscheidbareren Schallemission führt. So kann vorteilhaft die Präzision der Vorrichtung erhöht werden.

Gemäß einer bevorzugten Ausführungsform kann die Vorrichtung eingerichtet sein, eine Referenzmessung in einem Zustand, in dem der medizinische Artikel nicht an der medizinischen Vorrichtung angeordnet ist oder der medizinische Artikel an der medizinischen Vorrichtung angeordnet ist aber nicht angeregt wird, durchzuführen, wobei die Analyse auf Basis der Referenzmessung erfolgt.

Für die Referenzmessung kann beispielsweise lediglich die Schallemission erfasst werden, ohne dass eine Anregung erfolgt. Für die Referenzmessung kann beispielsweise der Artikel an der medizinischen Vorrichtung angeordnet sein. Für die Referenzmessung kann beispielsweise der medizinische Artikel noch nicht an der medizinischen Vorrichtung angeordnet sein. Die Referenzmessung bzw. die erfasste Schallemission der Referenzmessung kann für die Analyse der Schallemission bei der eigentlichen Messung dann von dieser abgezogen werden. Durch die Referenzmessung können vorteilhaft Umwelteinflüsse wie Vibrationen, Geräusche von anderen Maschinen berücksichtigt werden.

Gemäß einer bevorzugten Ausführungsform kann die Vorrichtung eine optische Erkennungseinheit aufweisen, die eingerichtet ist, den medizinischen Artikel zu erkennen und die Vorrichtung kann eingerichtet sein, eine Plausibilitätsprüfung durchzuführen.

Die optische Erkennungseinheit kann ein Photodetektor, eine Kamera, eine Barcode Reader oder dergleichen umfassen. Der medizinische Artikel kann eine Codierung, beispielsweise einen Strichcode, eine Farbcodierung oder dergleichen aufweisen. Die optische Erkennungseinheit kann in einer Datenbank zumindest eine Codierung für einen vorbestimmten medizinischen Artikel abgelegt haben. Durch einen Vergleich mit diesem kann die optische Erkennungseinheit den medizinischen Artikel dann erkennen. Die Vorrichtung kann vorteilhaft über zwei Messungen, die vibro-akustische Messung und die optische Messung den medizinischen Artikel identifizieren. Die Vorrichtung kann vorteilhaft über beide Messungen eine Plausibilitätsprüfung durchführen. Durch die Plausibilitätsprüfung kann vorteilhaft die Verlässlichkeit der Vorrichtung erhöht werden.

**In** anderen Worten kann die aus dem Stand der Technik bekannte medizinische Vorrichtung, insbesondere Infusionspumpe, welche ein Klemmenmodul zur Aufnahme einer als Schiebeklemme ausgebildeten Schlauchklemme aufweist, welches einen Photodetektor zur Erkennung der Farbe der Schiebeklemme aufweist, offenbarungsgemäß auch derart weitergebildet werden, dass sie neben der optischen Farberkennung noch die offenbarungsgemäße vibro-akustische Messung durchführt, was eine Plausibilitätsprüfung ermöglicht. Selbstverständlich kann die offenbarungsgemäße medizinische Vorrichtung alternativ nur für die offenbarungsgemäße vibro-akustische Messung eingerichtet sein, das heißt nicht für die optische Messung bzw. Farberkennung.

Gemäß einer bevorzugten Ausführungsform kann die Vorrichtung eine Infusionspumpe sein.

Gemäß einer bevorzugten Ausführungsform kann der medizinische Artikel eine Schiebeklemme eines Infusionsleitungssystems bzw. Schlauchsets sein oder umfassen.

Gemäß einer bevorzugten Ausführungsform kann die Schiebeklemme eine Farbkodierung umfassen, und kann die optische Erkennungseinheit eingerichtet sein, die Farbkodierung der Schiebeklemme zu erkennen.

Ein weiterer Aspekt der vorliegenden Offenbarung betrifft ein Verfahren zur Erkennung eines medizinischen Artikels, vorzugsweise mit einer oben näher beschriebenen Vorrichtung, wobei das Verfahren die Schritte umfasst: Anregen eines medizinischen Artikels, der sich an einer medizinischen Vorrichtung befindet, mit einer Anregung, sodass dieser in Schwingung versetzt wird; Erfassen einer Schallemission des angeregten medizinischen Artikels; Erkennen und/oder Klassifizieren des medizinischen Artikels durch Analyse der Schallemission, vorzugsweise Erkennen, ob der medizinische Artikel mit zumindest einem vorbestimmten medizinischen Artikel übereinstimmt, und vorzugsweise Ableiten eines Ergebnisses sowie Bereitstellen des Ergebnisses.

Das Ergebnis kann beispielsweise einem Backend oder einer Anzeige der medizinischen Vorrichtung bereitgestellt werden. Das Ergebnis kann einen Warnhinweis umfassen, wenn der medizinische Artikel nicht mit einem vorbestimmten medizinischen Artikel übereinstimmt.

Gemäß einer bevorzugten Ausführungsform kann das Verfahren weiter folgende Schritte umfassen: Durchführen einer Referenzmessung in einem Zustand, in dem der medizinische Artikel nicht an der medizinischen Vorrichtung angeordnet ist oder der medizinische Artikel in der medizinischen Vorrichtung angeordnet ist aber nicht angeregt wird, wobei die Analyse auf Basis der Referenzmessung erfolgt.

Die Referenzmessung kann beispielsweise von der erfassten Schallemission im Rahmen der Analyse abzogen werden, um Umwelteinflüsse zu minimieren.

Die Einheiten gemäß einem oder mehreren Ausführungsbeispielen können unter Verwendung von Hardware, Software und/oder einer Kombination davon implementiert werden. Die Einheiten können einteilig oder mehrteilig sein. Hardware-Einheiten können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung implementiert werden, die in der Lage ist, auf Befehle zu reagieren und diese in einer festgelegten Weise auszuführen.

Die Einheiten können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität einer bestimmten Einheit der vorliegenden Offenbarung kann auf mehrere Einheiten verteilt werden, die über Schnittstellenschaltungen verbunden sind.

Die Einheiten gemäß einem oder mehreren Ausführungsbeispielen können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND Flash) und/oder jeder andere Datenspeichermechanismus, der Daten speichern und aufzeichnen kann. Die eine oder mehrere Speichervorrichtungen können zum Speichern von Computerprogrammen, Programmcode, Anweisungen oder eine Kombination davon eingerichtet sein.

Die hier beschriebenen Erläuterungen und Vorteile einzelner Ausführungsformen gelten sinngemäß auch für die anderen Ausführungsformen. Verschiedene beispielhafte Merkmale der Ausführungsformen können offenbarungsgemäß kombiniert werden, wo immer dies technisch sinnvoll und machbar ist.

Die vorliegende Offenbarung wird nachfolgend mit Hilfe von Figuren erläutert. Es zeigen:
- Figur 1: eine schematische Ansicht einer Vorrichtung, die zur Erkennung eines medizinischen Artikels eingerichtet ist,
- Figur 2: eine Anregung eines medizinischen Artikels mit einer Frequenz,
- Figur 3: eine mechanische Anregung eines medizinischen Artikels,
- Figur 4: eine Anregung eines medizinischen Artikels mit mehreren Frequenzen,
- Figur 5: eine Anregung eines medizinischen Artikels mit einem Dirac Impuls,
- Figur 6: eine mechanische Anregung eines Artikels,
- Figur 7: eine Anregung eines medizinischen Artikels mit einem breitbandigen Signal,
- Figur 8: eine Vorrichtung mit einem medizinischen Artikel,
- Figur 9: einen exemplarischen Versuchsaufbau,
- Figur 10: unterschiedliche Messobjekte,
- Figur 11: eine Anregung für die Messobjekte,
- Figur 12: eine aufgenommene Schallemission,
- Figur 13: eine aufgenommene Schallemission,
- Figur 14: eine aufgenommene Schallemission,
- Figur 15: eine aufgenommene Schallemission, und
- Figur 16: eine aufgenommene Schallemission.

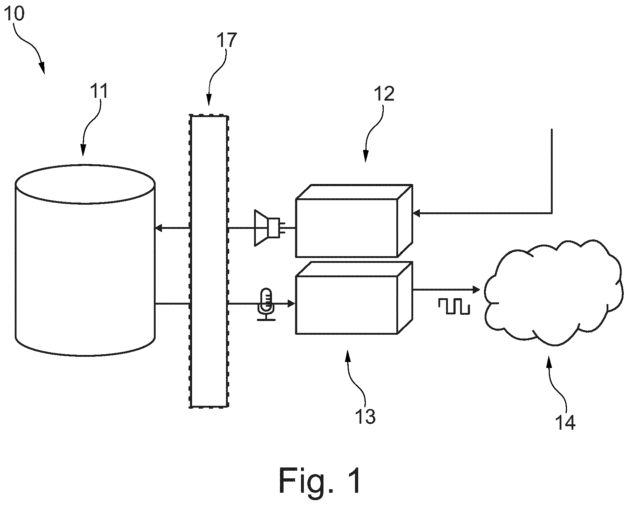

Figur 1 zeigt eine medizinische Vorrichtung 10, die zur Erkennung eines medizinischen Artikels 11 eingerichtet ist. Der medizinische Artikel 11 ist beispielsweise eine Schiebeklemme eines Infusionsleitungssystems. Die medizinische Vorrichtung 10 ist beispielsweise eine Infusionspumpe, in die die Schiebeklemme eingesetzt wird. Die medizinische Vorrichtung 10 umfasst eine Anregungseinheit 12, die eingerichtet ist, über eine Anregung den medizinischen Artikel 11 in Schwingung zu versetzen. Die Anregungseinheit 12 ist vorliegend ein Vibrationsmotor, der über ein Koppelelement 17 mit dem medizinischen Artikel 11 in Berührkontakt steht. Die Vorrichtung 10 umfasst weiter eine Erfassungseinheit 13, die eingerichtet ist, die Schallemission in Folge der Anregung, zu erfassen. Die Erfassungseinheit 13 ist vorliegend ein Mikrofon. Alternativ könnte die Erfassungseinheit 13 ein Beschleunigungssensor sein, der über das Koppelelement 17 mit dem medizinischen Artikel 11 verbunden ist. Die Vorrichtung 10 umfasst weiter eine Auswerteeinheit 14, die eingerichtet ist, durch eine Analyse der Schallemission den medizinischen Artikel zu erkennen und/oder zu klassifizieren und/oder seine Präsenz zu erkennen. Die Analyse kann dabei vorzugsweise einen Vergleich der erfassten Schallemission mit einem Klassifizierungsmuster für einen vorbestimmten medizinischen Artikel umfassen. Die Analyse kann vorzugsweise eine Auswertung einer oder mehrerer Frequenzen der Schallemission umfassen. Die Analyse kann vorzugsweise eine Auswertung einer oder mehrerer Amplituden der Schallemission umfassen. Die Analyse kann vorzugsweise die Auswertung einer oder mehrerer Phasenverschiebungen umfassen. Die Analyse kann vorzugsweise auf Basis eines Machine Learning Models erfolgen, das für einen vorbestimmten medizinischen Artikel trainiert wurde.

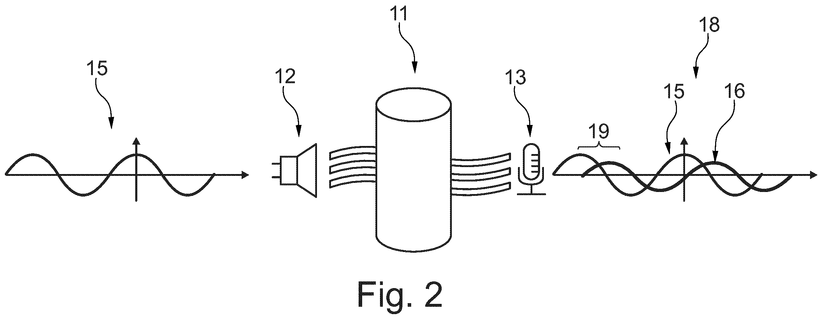

Figur 2 zeigt eine Anregung 15 eines medizinischen Artikels 11 mit einer Frequenz. Die Schallemission 16 und die Anregung 15 sind in einem Frequenzgang 18 aufgetragen. Es zeigt sich eine Phasenverschiebung 19 zwischen der Anregung 15 und der Schallemission 16. Die Analyse kann beispielsweise die Auswertung einer solchen Phasenverschiebung 19 und/oder der Amplitude und den Vergleich dieser mit einer Phasenverschiebung und/oder einer Amplitude eines Klassifizierungsmusters umfassen.

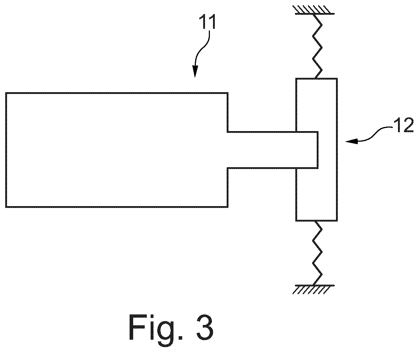

Figur 3 zeigt eine mechanische Anregung eines medizinischen Artikels 11 mit einem elastisch gelagerten Vibrationsmotor als Anregungseinheit 12.

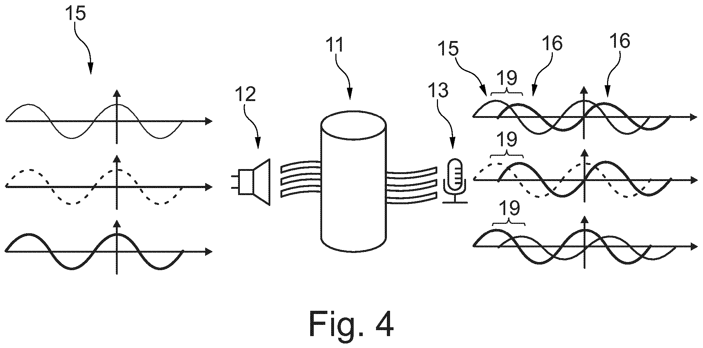

Figur 4 zeigt eine Anregung 15 eines medizinischen Artikels 11 mit drei unterschiedlichen Frequenzen sowie die zugehörigen Frequenzgänge 18 mit den unterschiedlichen Phasenverschiebungen 19 und Amplituden. Durch die Anregung des medizinischen Artikels 11 mit drei Frequenzen kann die Genauigkeit der Erkennung erhöht werden.

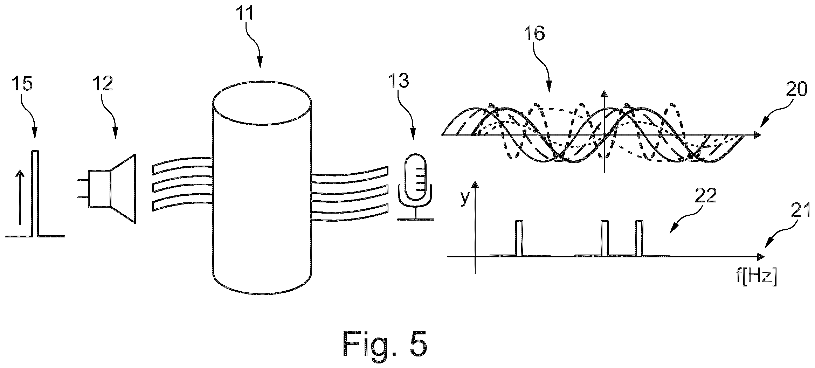

Figur 5 zeigt eine Anregung 15 eines medizinischen Artikels 11 mit einem Dirac Impuls. Die Schallemission 16 ist im oberen Diagramm über die Zeit 20 und im unteren Diagramm, Frequenzspektrum, über die Frequenz 21 aufgetragen. Der Dirac Impuls regt eine Vielzahl an Frequenzen des medizinischen Artikels 11 an. Im Frequenzspektrum 22 zeigen sich unterschiedliche Eigenfrequenzen der Schallemission, die im Rahmen der Analyse mit dem Klassifizierungsmuster zur Erkennung des medizinischen Artikels verglichen werden können.

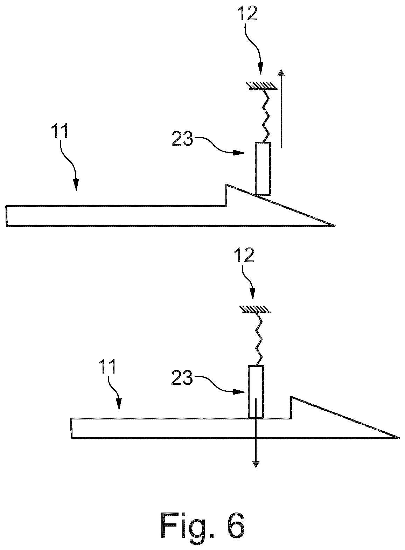

Figur 6 zeigt eine mechanische Anregung eines medizinischen Artikels 11 durch eine Anregungseinheit 12. Der medizinische Artikel 11 weist hierzu eine Rampe auf, die beim Einschieben des medizinischen Artikels 11 in die Vorrichtung 10 einen federgelagerten Stößel 23 zunächst vorspannt und dann gegen den medizinischen Artikel 11 prallen lässt. Dadurch kann beispielsweise einfach ein Dirac Impuls zur Anregung in den medizinischen Artikel 11 eingeleitet werden.

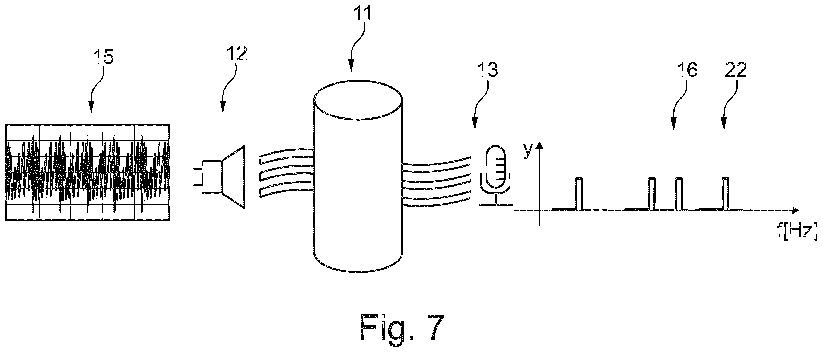

Figur 7 zeigt eine Anregung 15 eines medizinischen Artikels 11 mit einem breitbandigen Signal. Die Anregung 15 kann beispielsweise mit einem Lautsprecher und einem entsprechenden Signal erfolgen. Die Schallemission 16 ist als Frequenzspektrum 22 mit einer Vielzahl an charakteristischen Eigenfrequenzen dargestellt. Die Eigenfrequenzen können mit einem Klassifizierungsmuster zum Erkennen des medizinischen Artikels verglichen werden.

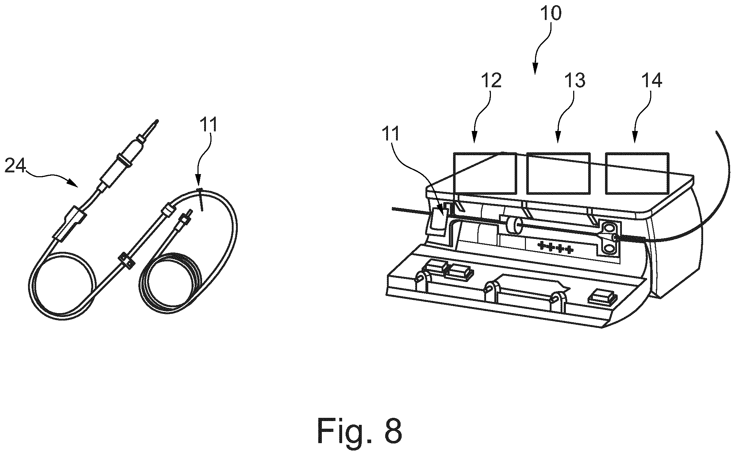

Figur 8 zeigt eine medizinische Vorrichtung 10. Die medizinische Vorrichtung 10 ist vorliegend eine Infusionspumpe. Der medizinische Artikel 11 ist vorliegend eine Schiebeklemme eines Schlauchsets 24 für die Infusionspumpe. Der medizinische Artikel 11 ist rechts im Bild in ein Klemmenmodul der Infusionspumpe eingesetzt und kann unter bestimmten Voraussetzungen einen Schlauch des Schlauchsets abklemmen oder einen Fluidfluss durch den Schlauch freigeben. Die Anregungseinheit 12, die Erfassungseinheit 13 und die Auswerteeinheit 14 sind vorliegend lediglich schematisch angedeutet. In dem gezeigten Ausführungsbeispiel kann optional zusätzlich eine optische Farberkennung der Schiebeklemme über eine optische Erkennungseinheit vorgenommen werden.

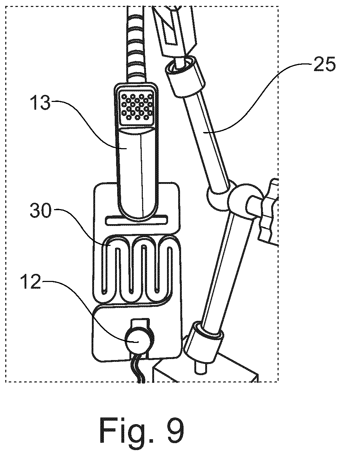

Figur 9 zeigt einen exemplarischen Versuchsaufbau. Der exemplarische Versuchsaufbau umfasst einen Ständer 25, an dem das Messobjekt 30 aufgehängt ist. Über eine Erfassungseinheit 13, vorliegend ein Körperschallaufnehmer, wird die Schallemission aufgenommen. Diese wird an einem Oszilloskop angezeigt. Die Anregung erfolgt mittels einer Anregungseinheit 12, vorliegend ein Vibrationsmotor. Der Vibrationsmotor wird von einem Treiber Breakoutboard und einem Arduino Microcontroller angesteuert. Das Messobjekt 30 ist vorliegend eine aus Kunststoff gedruckte Struktur mit einer horizontalen Meanderform.

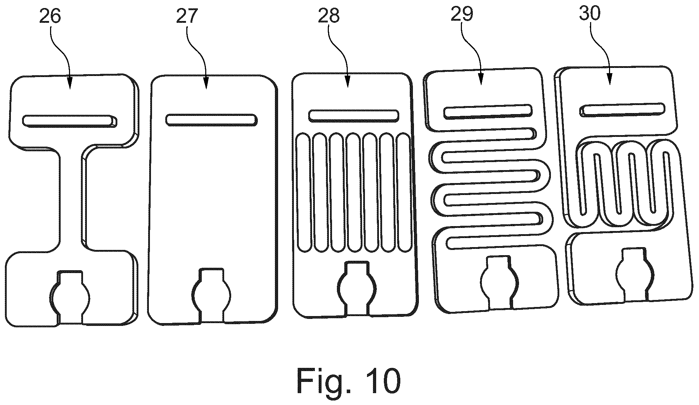

Figur 10 zeigt unterschiedliche Messobjekte 26 bis 30, die untersucht wurden. Messobjekt 26 ist eine aus Kunststoff gedruckte Struktur mit einem Steg. Messobjekt 27 ist eine aus Kunststoff gedruckte Struktur mit einer Platte. Messobjekt 28 ist eine aus Kunststoff gedruckte Struktur mit einem Gitter. Messobjekt 29 ist eine aus Kunststoff gedruckte Struktur mit einer vertikalen Meanderform. Messobjekt 30 ist eine aus Kunststoff gedruckte Struktur mit einer horizontalen Meanderform.

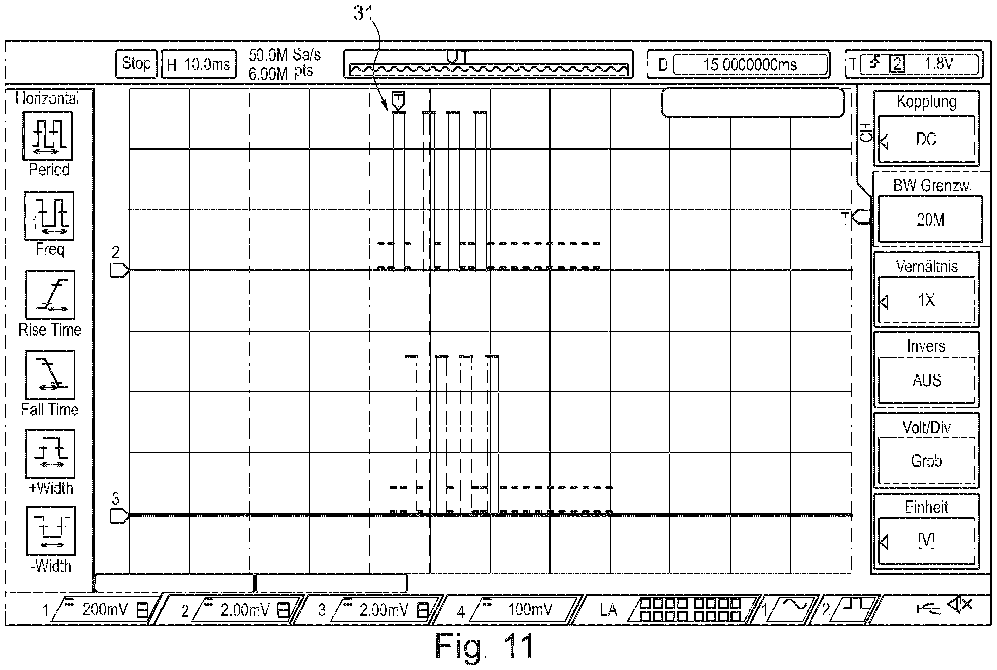

Figur 11 zeigt die Anregung 31 für die Messobjekte 26 bis 30. Die Anregung 31 ist für alle Messobjekte 26 bis 30 gleich.

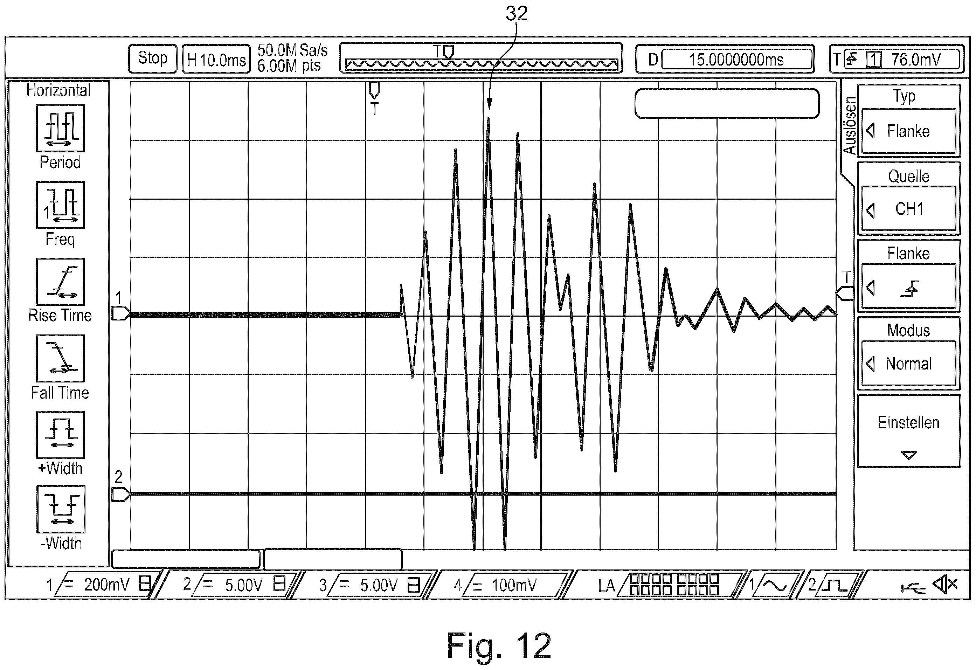

Figur 12 zeigt die aufgenommene Schallemission 32 für das Messobjekt 26.

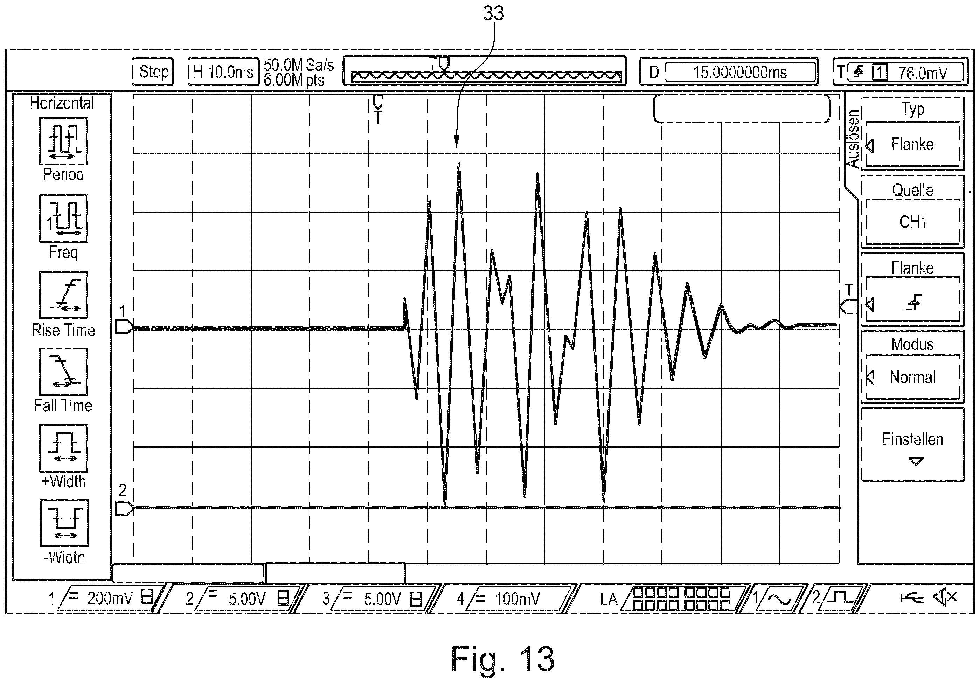

Figur 13 zeigt die aufgenommene Schallemission 33 für das Messobjekt 27.

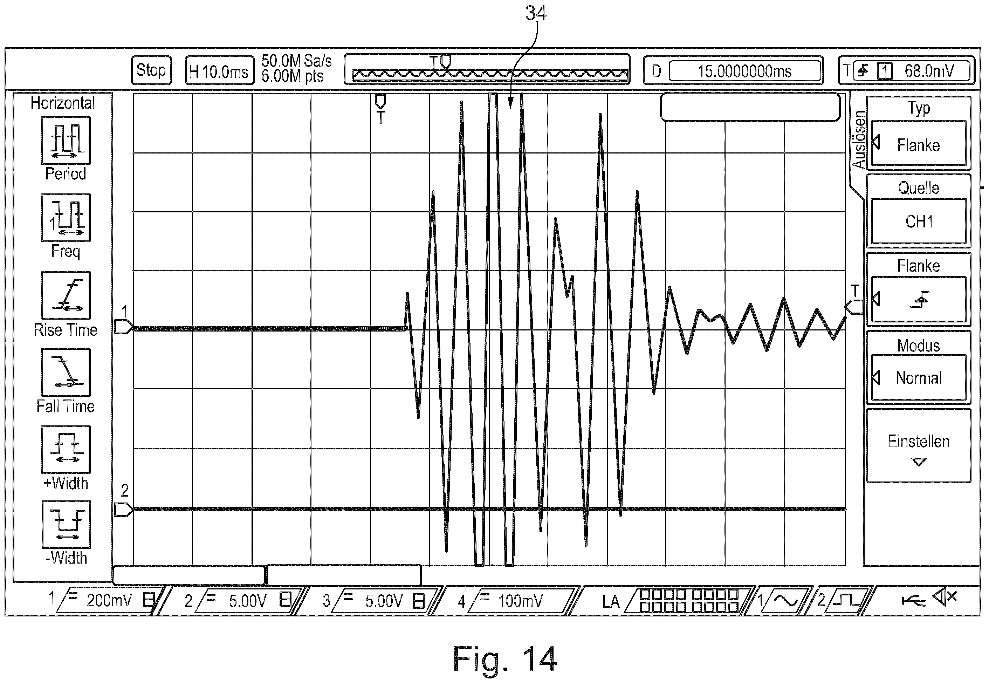

Figur 14 zeigt die aufgenommene Schallemission 34 für das Messobjekt 28.

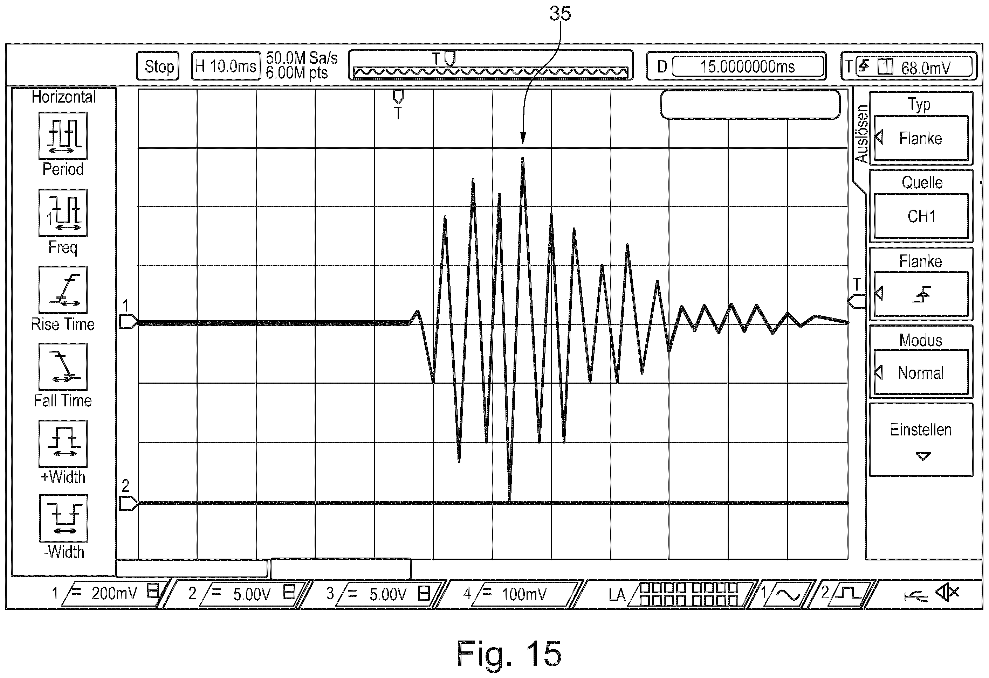

Figur 15 zeigt die aufgenommene Schallemission 35 für das Messobjekt 29.

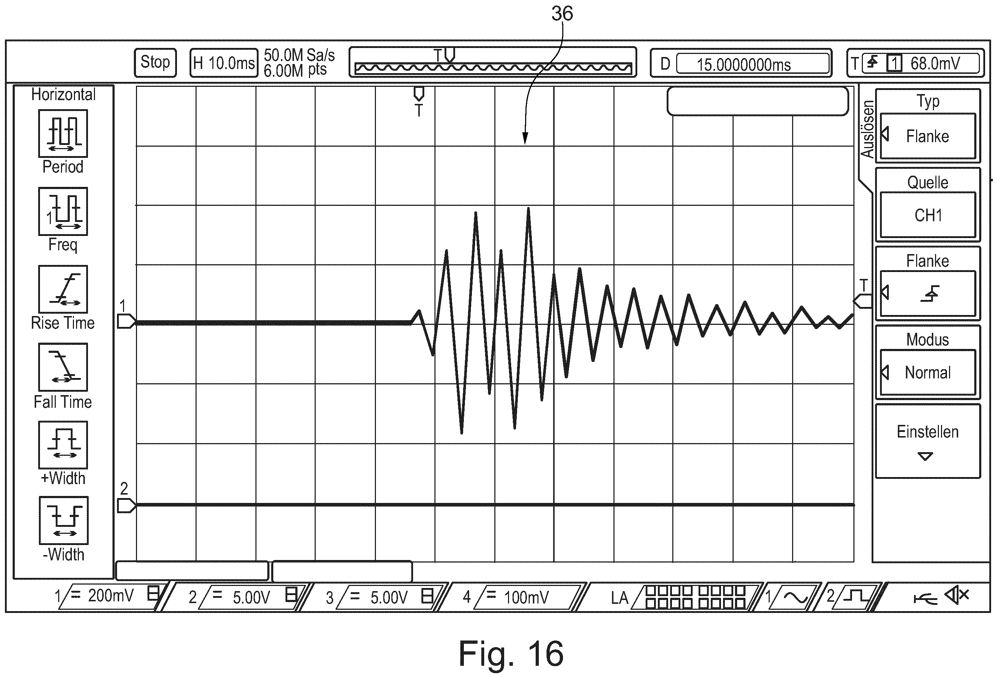

Figur 16 zeigt die aufgenommene Schallemission 36 für das Messobjekt 30.

Die Schallemissionen 32 bis 36 unterscheiden sich deutlich voneinander hinsichtlich des Amplitudenverlaufs bzw. der Amplitudenantwort. Somit ist eine Zuordnung der einzelnen Messobjekte 26 bis 30 auf Basis der Amplitudenantwort durch Auswertung von Amplitude, Amplitudendämpfung und Phase im Zeitbereich in Bezug auf die Anregung möglich. Weiterhin kann über eine Fourier Transformation die Analyse der Eigenfrequenzen eine weitere Möglichkeit der Zuordnung ermöglichen. Hier werden dann die Eigenfrequenzen miteinander verglichen.

### Bezugszeichenliste

- 10: Medizinische Vorrichtung
- 11: Medizinischer Artikel
- 12: Anregungseinheit
- 13: Erfassungseinheit
- 14: Auswerteeinheit
- 15: Anregung
- 16: Schallemission
- 17: Koppelelement
- 18: Frequenzgang
- 19: Phasenverschiebung
- 20: Zeit
- 21: Frequenz
- 22: Frequenzspektrum
- 23: federgelagerter Stößel
- 24: Schlauchset
- 25: Ständer für Versuch
- 26, 27, 28, 29, 30: Messobjekt
- 31: Anregung Messobjekte
- 32, 33, 34, 35, 36: Schallemission

## Patentansprüche

1. Medizinische Vorrichtung (10), die zur Erkennung, insbesondere Präsenzerkennung, und/oder Klassifizierung eines medizinischen Artikels (11) eingerichtet ist, umfassend:
eine Anregungseinheit (12), die eingerichtet ist, einen medizinischen Artikel (11) mit zumindest einer Anregung anzuregen, sodass dieser in Schwingung versetzt wird,
eine Erfassungseinheit (13), die eingerichtet ist, eine Schallemission (16) des angeregten medizinischen Artikels zu erfassen, und
eine Auswerteeinheit (14), die eingerichtet ist, durch eine Analyse der Schallemission (16) den medizinischen Artikel (11) zu erkennen und/oder zu klassifizieren, vorzugsweise zu erkennen, ob der medizinische Artikel (11) mit zumindest einem vorbestimmten medizinischen Artikel übereinstimmt.

2. Vorrichtung nach Anspruch 1,
wobei die Analyse eine Auswertung einer oder mehrerer der folgenden Größen umfasst: eine oder mehrere Frequenzen, eine oder mehrere Amplituden, eine oder mehrere Phasenverschiebungen.

3. Vorrichtung nach Anspruch 1 oder 2,
wobei die Analyse einen Vergleich mit zumindest einem Klassifizierungsmuster des zumindest einen vorbestimmten medizinischen Artikels umfasst.

4. Vorrichtung nach einem der vorherigen Ansprüche,
wobei die Analyse auf Basis eines Machine Learning Modells erfolgt,
wobei das Machine Learning Model für den zumindest einen vorbestimmten medizinischen Artikel trainiert wurde.

5. Vorrichtung nach einem der vorherigen Ansprüche,
wobei die Anregungseinheit eingerichtet ist, den medizinischen Artikel mechanisch anzuregen und/oder akustisch anzuregen.

6. Vorrichtung nach einem der vorherigen Ansprüche,
wobei die Anregungseinheit eine oder mehrere der folgenden Komponenten umfasst: Vibrationsmotor für Schwingungsanregung, Aktuator für Stoßanregung, Lautsprecher für akustische Anregung.

7. Vorrichtung nach einem der vorherigen Ansprüche,
wobei die Anregungseinheit eingerichtet ist, den medizinischen Artikel mit einem oder mehreren der folgenden Signale anzuregen: Dirac Impuls, eine Frequenz, mehrere Frequenzen, breitbandiges Frequenzsignal.

8. Vorrichtung nach einem der vorherigen Ansprüche,
wobei die Vorrichtung eingerichtet ist, eine Referenzmessung in einem Zustand, in dem der medizinische Artikel nicht an der medizinischen Vorrichtung angeordnet ist und/oder der medizinische Artikel in der medizinischen Vorrichtung angeordnet ist aber nicht angeregt wird, durchzuführen,
wobei die Analyse auf Basis der Referenzmessung oder Referenzmessungen erfolgt.

9. Vorrichtung nach einem der vorherigen Ansprüche,
wobei die Vorrichtung eine optische Erkennungseinheit aufweist, die eingerichtet ist, den medizinischen Artikel zu erkennen und wobei die Vorrichtung eingerichtet ist, eine Plausibilitätsprüfung durchzuführen.

10. Vorrichtung nach einem der vorherigen Ansprüche,
wobei die Vorrichtung eine Infusionspumpe ist.

11. Vorrichtung nach einem der vorherigen Ansprüche,
wobei der medizinische Artikel eine Schiebeklemme eines Schlauchsets ist oder umfasst.

12. Vorrichtung nach den Ansprüchen 9 bis 11,
wobei die Schiebeklemme eine Farbkodierung umfasst, und wobei die optische Erkennungseinheit eingerichtet ist, die Farbkodierung zu der Schiebeklemme zu erkennen.

13. Verfahren zur Erkennung eines medizinischen Artikels, vorzugsweise mit einer Vorrichtung nach einem der Ansprüche 1 bis 12, wobei das Verfahren die Schritte umfasst:
Anregen eines medizinischen Artikels, der sich an einer medizinischen Vorrichtung befindet, mit einer Anregung, sodass dieser in Schwingung versetzt wird,
Erfassen einer Schallemission des angeregten medizinischen Artikels, und
Erkennen und/oder Klassifizieren des medizinischen Artikels durch Analyse der Schallemission, vorzugsweise Erkennen, ob der medizinische Artikel mit zumindest einem vorbestimmten medizinischen Artikel übereinstimmt.

14. Verfahren nach Anspruch 13, ferner mit dem Schritt:
Durchführen einer Referenzmessung in einem Zustand, in dem der medizinische Artikel nicht an der medizinischen Vorrichtung angeordnet ist und/oder der medizinische Artikel in der medizinischen Vorrichtung angeordnet ist aber nicht angeregt wird,
wobei die Analyse auf Basis der Referenzmessung oder Referenzmessungen erfolgt.

15. Verfahren nach Anspruch 13 oder 14, ferner mit dem Schritt:
Optische Erkennung des medizinischen Artikels zur Durchführung einer Plausibilitätsprüfung.
